# FASCICULE DE BREVET EUROPEEN

(11) **EP 0 861 252 B1**
(45) Date de publication et mention de la délivrance du brevet: **23.02.2000**
(21) Numéro de dépôt: 96931105.9
(22) Date de dépôt: 11.09.1996
(51) Int. Cl.: C07D 451/04, A61K 31/435, C07D 451/14

(54) **DERIVES NAPHTAMIDE DE 3-BETA-AMINO AZABICYCLO OCTANE OU NONANE, COMME AGENTS ANTIPSYCHOTIQUES**
NAPHTHAMIDDERIVATE VON 3-BETA-AMINO AZABICYCLOOCTAN ODER NONAN ALS ANTIPSYCHOTISCHE ARZNEIMITTEL
NAPHTHAMIDE DERIVATIVES OF 3 BETA-AMINO AZABICYCLO OCTANE OR NONANE AS NEUROLEPTIC AGENTS

(30) Priorité: 12.09.1995 FR 9510655
(43) Date de publication de la demande: 02.09.1998
(73) Titulaire: PIERRE FABRE MEDICAMENT, 92100 Boulogne-Billancourt (FR)
(72) Inventeur: IMBERT, Thierry, F-81290 Viviers-les-Montagnes (FR); MONSE, Barbara, F-81100 Castres (FR); KOEK, Wouter, F-81290 Viviers-les-Montagnes (FR)
(74) Mandataire: Ahner, Francis
(86) Numéro de dépôt international: FR9601394
(87) Numéro de publication internationale: WO9710244

(56) Documents cités:
- EP-A- 0 076 592
- EP-A- 0 539 281
- EP-A- 0 585 116
- US-A- 5 395 835

## Description

La présente invention concerne de nouveaux dérivés azabicyclo-naphtalinecarboxamides, leur procédé de préparation et leur utilisation comme médicament.

Ces composés sont des agents antidopaminergiques et antisérotoninergiques et utilisés ainsi en tant que médicament antipsychotique pour traiter la schizophrénie, ses symptômes positifs et négatifs, ou les troubles du système nerveux central sensibles au traitement antidopaminergique et antisérotoninergique comme, par exemple, les désordres obsessionnels compulsifs, l'anxiété, la dépression, la toxicomanie, de la dyskinésie tardive et les troubles gastro-intestinaux.

La nécessité d'avoir une activité antidopaminergique, en particulier sur les récepteurs dopaminergiques de la sous-classe D₂, constitue une approche classique du traitement de la schizophrénie (Carlsson A., Am. J. Psychiatry, **135**, 164, 1978). Cependant, la plupart des composés ayant un tel mécanisme d'action ont l'inconvénient de présenter en clinique des effets secondaires indésirables, en particulier d'ordre extrapyramidaux (voir Acta Psychiatr. Scand. 1995, **91** (Suppl 388) : 24-30).

Il est apparu que le fait d'associer à une puissante activité antidopaminergique, dans la même molécule, une activité antagoniste des récepteurs sérotoninergiques de la sous-classe 5-HT₂ pourrait constituer une condition pour éviter ces effets extrapyramidaux secondaires (H. Y. Meltzer J.P.E.T. 1989, **251**, 238).

Le brevet US 5,395,835 décrit des naphtamides de formule 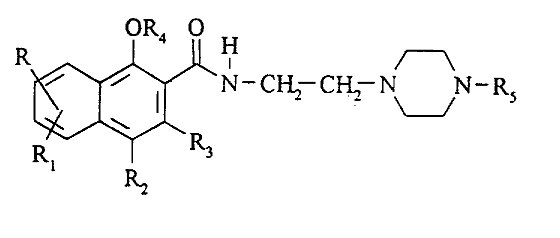 où le noyau naphtalène est diversement substitué par des groupements R variables, ces produits sont des agents antidopaminergiques de la sous-classe D₃, utiles comme agents antipsychotiques pour le traitement de la schizophrénie ou d'autres pathologies qui répondent au blocage des récepteurs dopaminergiques

Le brevet EP 539281 décrit des naphtamides de formule 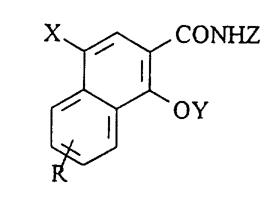 où Z représente un reste provenant de 2-aminométhyl N-alkyl pyrrolidine, 2-aminoéthyl-N,N-diéthylamine, 2-aminoéthyl morpholine, 2-aminoéthyl-N,N-dibutylamine, 4-amino-N-butyl (ou N-Benzyl) pipéridine, actifs sur le système dopaminergique, en particulier la sous-classe des récepteurs D₃ et utiles comme agents antipsychotiques, psychostimulants, antiautistiques, antidépresseurs, antiparkinsoniens, antihypertenseurs. Le composé le plus proche structurellement des composés de la présente invention correspond à la structure ci-dessus où R = H, Y = CH₃, X= H, Z = 4-amino N-benzylpiperidine , et sera appelé dans ce qui suit: composé brevet EP 539281.

Le brevet EP 585116 décrit des l-alcoxynaphtalène-2-carboxamides, avec une haute affinité sur les récepteurs sérotoninergiques 5-HT_{1A}, de formule

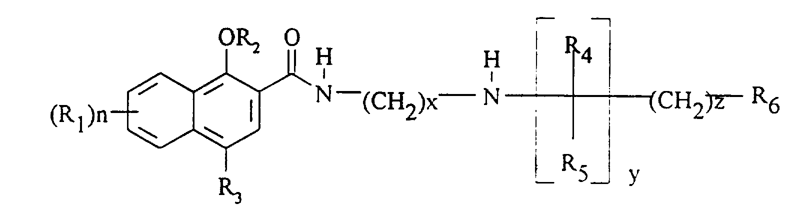

Le brevet US 4,536,580 décrit des dérivés benzamide du nortropane, ayant des propriétés neuroleptiques, de formule 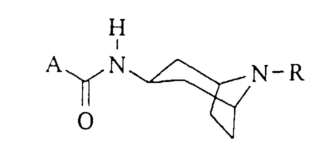 où A représente un noyau pyrimidinique ou benzénique différemment substitué, le produit le plus puissant étant le composé n° 64 (tropapride) (Drug of the future, vol. 9, n° 9, 673).

Le brevet EP 416521 décrit des composés du type naphtamide tropane, de structure : 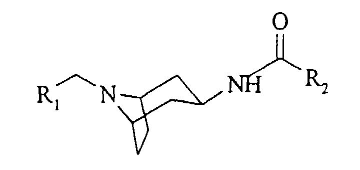 où R₂ peut être un noyau naphtalène, soit non substitué soit substitué par un groupement alkyl de 1 à 10 atomes de carbone. Ces composés sont actifs dans le domaine cardiovasculaire.

Le brevet WO 84/03281 décrit des composés de type azabicycloalkyl benzamides de formule 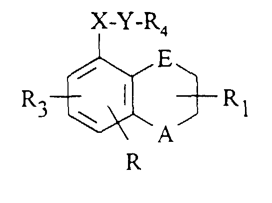 où R₄ peut être un squelette tropane et le cycle formé par la jonction A-E peut être un hétérocycle et former une quinoléine.

Cependant, il n'apparaît pas, ou n'est pas suggéré dans les brevets sus mentionnés, que le fait d'associer un squelette 3β aminotropane à un système aromatique 1-alcoxy 2-naphtamide, puisse fournir des composés ayant cette double activité : à la fois antagoniste et agoniste dopaminergique sur la sous-classe D₂, et à la fois antagoniste et agoniste sérotoninergique ce qui est le fait de la présente invention.

La meilleure balance que représente les dérivés de l'invention entre les récepteurs sérotoninergiques, en particulier mais non exclusivement les récepteurs 5-HT₂, en comparaison des récepteurs dopaminergiques D₂, permet de mettre en lumière l'avantage de ces produits en clinique humaine, alliant l'efficacité thérapeutique associée à une faible propention à manifester des effets secondaires extrapyramidaux (Meltzer H.Y. Psychopharmacology, 1989, 99, 18-27 et Meltzer H.Y. J. Clin. Psychopharmacology, 15 suppl. 1 : 1S, 1995).

Le meilleur composé actuellement utilisé et correspondant aux critères ainsi définis est la Rispéridone, de structure :

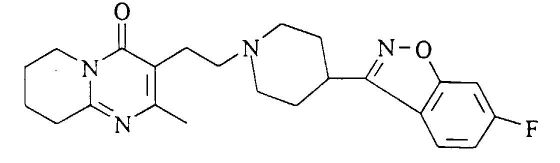

La présente invention a pour objet les nouveaux dérivés 1-alcoxy 2-naphtamide substitués de 3β-amino-tropane-8-benzyl substitué, leur procédé de préparation, leur forme de sel pharmaceutiquement acceptable, les compositions pharmaceutiques les contenant et leur application en tant que médicament en thérapeutique humaine comme antipsychotique.

Ces nouveaux composés répondent à la formule générale I 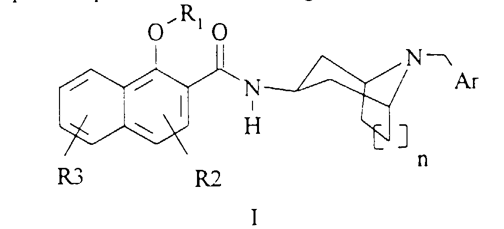 dans laquelle Ar représente un noyau phényl, le noyau phényl étant ou non substitué par un ou plusieurs substituants choisis parmi, alkyl C₁₋₄, halogène Cl, F, Br, O alkyl C₁₋₄,
n pouvant être un ou deux et forme ainsi un 8-azabicyclo[3.2.1]octane ou un 9-azabicyclo[3.3.1]nonane.
R₁ représente un groupe alkyl, linéaire ou ramifié en C₁₋₆.
R₂ et R₃ identiques ou différents, portés par les carbones du cycle aromatique, chacun pouvant être H, Cl, Br, F, alkyl en C₁₋₄, OH, CN, NO₂, alkylthio en C₁₋₄, NH₂, alkylamino en C₁₋₄, dialkylamino en C₁₋₄, acylamino, SO₂NH₂, SO₂Ndialkyl en C₁₋ ₄, SO₂alkyl en C₁₋₄ .

De manière préférentielle, le groupement Ar est un phényl substitué ou non par un halogène, le substituant R₁ peut être un méthyle ou un éthyle, le substituant R₂ en position 4 étant préférentiellement un H, Br, Cl, F, NO₂, NH₂,NMe₂, CN, OCH₃, OH et correspondent ainsi aux composés suivants :
◆ 1-Méthoxy-4-(N,N-diméthylaminosulfonyl)-N-[8-(phénylméthyl)-8-azabicyclo-[3.2.1]oct-3-β-yl]-2-naphthalène carboxamide
◆ 1,3-Diméthoxy-N-[8-(phénylméthyl)-8-azabicyclo[3.2.1]oct-3-b-yl]-2-naphthalène carboxamide
◆ 1-Méthoxy-N-[8-(phénylméthyl)-8-azabicyclo[3.2.1]oct-3-b-yl]-2-naphthalène carboxamide
◆ 4-Bromo-1-méthoxy-N-[8-(phénylméthyl)-8-azabicyclo[3.2.1]oct-3-b-yl]-2-naphthalène carboxamide
◆ 4-Chloro-1-méthoxy-N-[8-(phénylméthyl)-8-azabicyclo[3.2.1]oct-3-b-yl]-2-naphthalène carboxamide
◆ 1-Méthoxy-4-(N,N-diméthylamino)-N-[8-(phénylméthyl)-8-azabicyclo[3.2.1]oct-3-b-yl]-2-naphthalène carboxamide
◆ 4-Amino-1-méthoxy-N-[8-(phénylméthyl)-8-azabicyclo[3.2.1]oct-3-b-yl]-2-naphthalène carboxamide
◆ 1-Méthoxy-4-nitro-N-[8-(phénylméthyl)-8-azabicyclo[3.2.1]oct-3-b-yl]-2-naphthalène carboxamide
◆ 4-Cyano-1-méthoxy-N-[8-(phénylméthyl)-8-azabicyclo[3.2.1]oct-3-b-yl]-2-naphthalène carboxamide
◆ 1,5-Diméthoxy-N-[8-(phénylméthyl)-8-azabicyclo[3.2.1]oct-3-b-yl]-2-naphthalène carboxamide
◆ 1,4-Diméthoxy-N-[8-(phénylméthyl)-8-azabicyclo[3.2.1]oct-3-b-yl]-2-naphthalène carboxamide
◆ 1-Ethoxy-N-[8-(phénylméthyl)-8-azabicyclo[3.2.1]oct-3-b-yl]-2-naphthalène carboxamide
◆ 4-Bromo-1-éthoxy-N-[8-(phénylméthyl)-8-azabicyclo[3.2.1]oct-3-b-yl]-2-naphthalène carboxamide
◆ 4-Acétamido-1-méthoxy-N-[8-(phénylméthyl)-8-azabicyclo[3.2.1]oct-3-b-yl]-2-naphthalène carboxamide
◆ 4-Acétamido-1-méthoxy-N-[8-(4-fluorophenylmethyl)-8-azabicyclo[3.2.1]oct-3-b-yl]-2-naphthalène carboxamide
◆ 4-Acétamido-1-méthoxy-N-[8-(4-chlorophenylmethyl)-8-azabicyclo[3.2.1]oct-3-b-yl]-2-naphthalène carboxamide
◆ 4-Amino-1-méthoxy-N-[8-(4-chlorophenylmethyl)-8-azabicyclo[3.2.1]oct-3-b-yl]-2-naphthalène carboxamide
◆ 4-Amino-1-méthoxy-N-[8-(4-fluorophenylmethyl)-8-azabicyclo[3.2.1]oct-3-b-yl]-2-naphthalène carboxamide
◆ 4-Amino-1-éthoxy-N-[8-(4-fluorophenylmethyl)-8-azabicyclo[3.2.1]oct-3-b-yl]-2-naphthalène carboxamide
◆ 4-Fluoro-1-éthoxy-N-[8-(phénylméthyl)-8-azabicyclo[3.2.1]oct-3-b-yl]-2-naphthalène carboxamide
◆ 4-Fluoro-1-méthoxy-N-[8-(phénylméthyl)-8-azabicyclo[3.2.1]oct-3-b-yl]-2-naphthalène carboxamide
◆ 4-Bromo-1-méthoxy-N-[9-(phénylméthyl)-9-azabicyclo[3.3.1]non-3-b-yl]-2-naphthalène carboxamide
◆ 4-Amino-1-méthoxy-N-[9-(phénylméthyl)-9-azabicyclo[3.3.1]non-3-b-yl]-2-naphthalène carboxamide
◆ 4-Bromo-1-méthoxy-N-[8-(4-fluorophenylmethyl)-8-azabicyclo[3.2.1]oct-3-b-yl]-2-naphthalène carboxamide
◆ 4-Bromo-1-méthoxy-N-[8-(4-chlorophenylmethyl)-8-azabicyclo[3.2.1]oct-3-b-yl]-2-naphthalène carboxamide
◆ 4-Méthylthio-1-méthoxy-N-[8-(phénylméthyl)-8-azabicyclo[3.2.1]oct-3-b-yl]-2-naphthalène carboxamide
◆ 4-Ethylthio-1-méthoxy-N-[8-(phénylméthyl)-8-azabicyclo[3.2.1]oct-]-b-yl]-2-naphthalène carboxamide
◆ 4-Ethylsulfonyl-1-méthoxy-N-[8-(phénylméthyl)-8-azabicyclo[3.2.1]oct-3-b-yl]-2-naphthalène carboxamide
◆ 4-Aminosulfonyl-1-méthoxy-N-[8-(phénylméthyl)-8-azabicyclo[3.2.1]oct-3-b-yl]-2-naphthalène carboxamide
◆ 4-Hydroxy-1-méthoxy-N-[8-(phénylméthyl)-8-azabicyclo[3.2.1]oct-3-b-yl]-2-naphthalène carboxamide
◆ 4-Hydroxy-1-éthoxy-N-[8-(phénylméthyl)-8-azabicyclo[3.2.1]oct-3-b-yl]-2-naphthalène carboxamide
◆ 1-Méthoxy-N-[9-(phénylméthyl)-9-azabicyclo[3.3.1]non-3β-yl]-2-naphtalène carboxamide
◆ 1-Ethoxy-4-nitro-N-[8-(phénylméthyl)-8-azabicyclo[3.2.1]oct-3β-yl]-2-naphtalène carboxamide

Les composés de la présente invention peuvent former des sels par addition d'acide minéral ou organique pharmaceutiquement acceptable et entrer dans des compositions pharmaceutiques pour pouvoir être administrées par les différentes voies usuelles, forme orale, injectable, parentérale.

La mise en évidence des propriétés antidopaminergiques et antisérotoninergiques des composés de l'invention est faite sur la base de leur affinité sur les récepteurs correspondants par déplacement du ligand radioactif qui marquent spécifiquement ces récepteurs ([³H] YM-09151-2 pour le récepteur D₂ et [³H] Ketansérine pour le récepteur 5-HT₂).

Ce procédé d'étude de liaison spécifique est décrit Naunyn-Schmiedeberg's Arch. Pharmacol. 329, 333-338, 1985 et Mol. Pharmacol. 21, 301-314, 1982.

A titre d'exemple, les valeurs sont indiquées dans le tableau suivant en comparaison des substances de référence

| **Liaison au récepteur K**_{**i**} **(M)** | | |
|---|---|---|
| **Composé** | **Site D**_{**2**} **ligand [**^{**3**}**H] YM09151-2 Ki** | **Site 5-HT**_{**2**} **ligand [**^{**3**}**H] Ketansérin Ki** |
| Sulpiride | 4.63 10⁻⁹ M | >10⁻⁵ M |
| Risperidone | 2.00 10⁻⁹ M | 2.74 10⁻⁹ M |
| Tropapride US4,536,580 | 1.06 10⁻¹⁰ M | 2.94 10⁻⁷ M |
| Composé brevet EP 539281 | 3.32 10⁻⁹ M | 1.29 10⁻⁶ M |
| Exemple 3 | 1.43 10⁻¹⁰M | 6.68 10⁻⁸M |
| Exemple 5 | 4.08 10⁻¹⁰M | 1.64 10⁻⁸ M |
| Exemple 4 | 7.76 10⁻¹⁰M | 3.76 10⁻⁹ M |
| Exemple 13 | 9.44 10⁻¹⁰ M | 7.79 10⁻⁹ M |

Il ressort de cette étude que, de façon surprenante, les composés de l'invention de structure selon la formule 1, possèdent des affinités sur les deux récepteurs D₂ et 5-HT₂ comme on peut le voir dans ce tableau, comparativement avec les substances de référence : le Sulpiride composé antipsychotique usuellement utilisé, la Rispéridone composé antipsychotique atypique tel que défini par H. Y. Meltzer, le Tropapride (US 4536580), de même que le composé du brevet EP 539281, qui ne présentent que peu ou pas d'affinité 5-HT₂.

L'intérêt des composés de l'invention apparaît par la plus grande affinité, 10 fois à 100 fois plus, qu'ils manifestent sur les récepteurs sérotoninergiques 5-HT₂ par rapport au Tropapride ou au composé du brevet EP 539281 et possèdent une meilleure balance entre les récepteurs dopaminergiques et sérotoninergiques, ce qui permet d'obtenir des composés possédant un meilleur profil clinique, pour non seulement avoir la puissance des effets antipsychotiques mais aussi l'absence des effets secondaires indésirables.

Le test *in vivo,* pratiqué chez le rat, montrant l'activité antipsychotique, est celui de l'inhibition des comportements induits par le méthylphénidate selon la méthode décrite par W. KOEK et F.C. COLPAERT dans J. Pharmacol. Exp. Ther. 1993, 267, 181.

Les résultats obtenus ont montré que les composés de l'invention sont capables non seulement d'inhiber le machonnement stéréotypé, mais aussi de normaliser tous les comportements induits par le méthylphénidate et ceci en l'absence d'effets secondaires indésirables.

Ce test caractérise l'activité antipsychotique des composés de manière plus approfondie que l'antagonisme des effets de l'apomorphine, test classiquement utilisé.

La présente invention concerne donc les composés de formule générale I comme médicaments utiles en particulier dans le traitement de la schizophrénie.

La présente invention concerne également l'utilisation des composés de formule générale I entrant dans une composition pharmaceutique de formulation correspondante à son mode d'administration, comprimés, capsules, gélules adaptés à la clinique humaine et à des doses quotidiennes comprises entre 0,1 et 500 mg ou plus spécifiquement 0,1 à 100 mg de principe actif.

Les produits de la présente invention sont obtenus par des procédés connus dont l'étape clé est la formation de la fonction amide entre l'acide 1-alcoxy 2-naphtoïque de formule II et l'amine de formule III

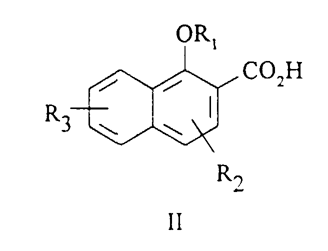

Les valeurs de R₁, R₂, R₃ sont les mêmes que celles de la formule générale I. Ces acides sont soit connus dans la littérature chimique soit préparés par les méthodes usuelles, par analogie avec les acides benzoïques substitués (du type salicylique).

Pour exemple, l'acide benzoique II
où R₁ = CH₃ ; R₂ = R₃ = H est décrit dans Monatshef. Chem. 1884, 15, 735 où R₁ = CH₃ ; R₂ = 4-NO₂ ; R₃ = H dans Indian Acad. Sci. Sect. A 1938, 7, 261 où R₁ = CH₃ ; R₂ = H ; R₃ = 5-OCH₃ est décrit dans J. Chem. Soc 1937, 937-940 où R₁ = C₂H₅ ; R₂ = R₃ = H est décrit dans Austr. J. Chem. 1974, 27, 2209 où R₁ = CH₃ ; R₂ = 4-Br ; R₃ = H est décrit dans J. Indian Chem. Soc. 1936, 13, 645.

De façon analogue, est préparé l'acide II où R₁ = C₂H₅ ; R₂ = 4-Br ; R₃ = H, dans le même article est préparé l'acide II où R₁ = CH₃ ; R₂ = 4-Cl ; R₃ = H, l'acide II où R₁ = CH₃ ; R₂ = 4-OH ; R₃ = H est décrit dans le J. Am. Chem. Soc. 1942, 64, 798-800 ; et où R₁ = CH₃ ; R₂ = 4-NH₂ ; R₃ = H, dans J. Chem. Soc. 1922, 1658, de même où R₁ = CH₃ ; R₂ = 4-OCH₃ ; R₃ = H, dans J. Organomet. Chem. 1969, 20, 251 ; où R₁ = CH₃ ; R₂ = 3-OCH₃ ; R₃ = H, dans J. Am. Chem. Soc. 1952, 74, 1624, où R₁ = CH₃ ; R₂ = 4-NMe₂ ; R₃ = H dans le brevet EP 585116. L'acide II où R₁ = CH₃ ; R₂ = 4-CN ; R₃ = H s'obtient par la réaction classique de Sandmeyer à partir du dérivé 4-amino décrit dans le brevet EP 539281.

L'acide II où R₁ = CH₃ ; R₂ = SCH₃ ; SC₂H₅, SO₂CH₃, SO₂C₂H₅, SO₂NH₂ s'obtient selon le procédé décrit dans les brevets Be 874490 ou Spain 454931 sur les acides benzoïques 2-alcoxy, par réaction avec la chlorhydrine sulfurique et pour obtenir le dérivé 4-SO₂Cl qui se réduit en se dimérisant en dérivé dithio, duquel on peut obtenir le dérivé méthylthio ou éthylthio par traitement par l'halogénure d'alkyl en milieu basique. Par oxydation, au moyen du permanganate de potassium ou à l'aide de peracides comme l'acide métachloroperbenzoïque, on peut obtenir les substituants sulfonyls correspondants.

L'amine polycyclique 3β-amino 8-azabicyclo[3.2.1]octane 8-benzyl de formule III (n = 1) est préparée selon le procédé décrit (Eur. J. Med. Chim. 1984, **19**, 105)

L'amine polycyclique 3β-amino 9-azabicyclo[3.3.1]nonane 9-benzyl de formule III (n = 2) est préparée par analogie directe et selon le procédé décrit dans J. Org. Chem. **26**, 395, 1961. 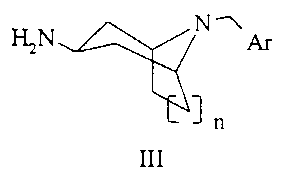 Ar a la même caractéristique que ce qui est mentionné pour la formule I.

La condensation de l'acide II et l'amine III se fait par activation de l'acide II soit par l'intermédiaire de son chlorure d'acide, par exemple le chlorure d'acide de l'acide naphtalénique correspondant, soit par l'intermédiaire anhydride mixte obtenu par réaction de l'acide II avec un chloroformiate d'alkyle par exemple d'éthyle à 0° C en présence d'une base comme la triéthylamine dans le chlorure de méthylène ou autre solvant inerte, suivi de la réaction avec l'amine de formule III.

Le dérivé NH₂ en position 4 peut être acétylé dans les conditions usuelles c'est-à-dire par l'anhydride acétique seul ou dans la pyridine ou bien par le chlorure d'acétyle dans le CH₂Cl₂ ou le THF en présence d'une base comme la triéthylamine ou le K₂CO₃.

La débenzylation de la fonction N-benzyl de l'hétérocycle peut s'effectuer en présence d'hydrogène sur charbon palladié à 10 % en milieu neutre ou acide, éventuellement sous pression en autoclave. Dans le cas des composés halogénés en postion 4, la méthode de déalkylation par le α-chloroethylchloroformate est utilisée comme indiqué dans J. Org. Chem. 1984, **49**, 2081.

L'alkylation de l'amine secondaire résultant par un chlorure de benzyle substitué se fait de façon classique au reflux de l'acétonitrile en présence éventuellement d'une base comme le K₂CO₃. La déacétylation de l'aniline en position 4 peut se faire par chauffage en milieu acide par exemple acide chlorhydrique aqueux dans un solvant miscible à l'eau comme l'éthanol.

La fluoration en position 4 peut se faire de façon également classique comme il est indiqué dans Organic Syntheses Coll. Vol. II, p. 299, par décomposition du tétrafluoroborate de diazonium sur l'ester naphtalénique correspondant

Les dérivés phénoliques en position 4 du noyau naphtalène s'obtiennent par hydrogénolyse des dérivés protégés par un groupement benzyle ou benzyl carbonate, par l'hydrogène en présence de charbon palladié à 10 % (T. N. Greene Protective Groups in Organic Synthesis 1991, p. 156 et 157) à partir des composés 1 où R₁ = CH₃ ; R₂ = OCH₂Ph ; R₃ = H ; dans le cas présent, la fonction N-benzyl n'est pas affectée par ces conditions d'hydrogénolyse.

L'acide II où R₁ = CH₃ ; R₂ = 4-OH ; R₃ = H connu est protégé par un des groupements protecteurs sus mentionnés avant l'étape d'amidification.

A titre d'exemples et de façon non limitative, les exemples suivants sont donnés :

### Exemple 1:

### 1-Méthoxy-4-(N,N-diméthylaminosulfonyl)-N-[8-(phénylméthyl)-8 azabicyclo[3.2.1]oct-3-β-yl]-2-naphthalène carboxamide chlorhydrate. (Formule I : R1 = CH₃ ; R2 = 4-SO₂N(CH₃)₂ ; R3 = H ; Ar = Ph ; n = 1)

### Stade 1. 1-Méthoxy-4-(N,N-diméthylaminosulfonyl)-2-naphthalène carboxylate de méthyle.

On chauffe à reflux un mélange de 2.0 g d'acide 4-aminosulfonyl-1-hydroxy-2-naphthoïque (7.48 mmole ; 1 eq) avec 3.0 ml de diméthylsulfate (32 mmole ; 4.2 eq) et 4.65 g de carbonate de potassium (33.7 mmole ; 4.5 eq) dans 100 ml d'acétone pendant la nuit. On filtre le précipité et on évapore le solvant sous vide. On reprend dans l'acétate d'éthyle, on lave plusieurs fois par l'eau, on sèche par Na₂SO₄ et on évapore sous vide.

On obtient 2.3 g du produit pur (95 %).
RMN (1H ; CDCl₃) : 2.79 (s ; 6H ; N-(CH₃)₂), 3.97 (s ; 3H ; C(O)OCH₃), 4.10 (s ; 3H; OCH₃) ; 7.59-7.77 (m ; 2H), 8.37 (dd, J = 7.7, 1.6 Hz ; 1H), 8.56 (s ; 1H), 8.70 (dd, J = 7.7, 1.6 Hz ; 1H).

### Stade 2 : Acide 1-méthoxy-4-(N,N-diméthylaminosulfonyl)-2-naphthalène carboxylique

On chauffe à reflux une solution de 1-méthoxy-4-(N,N-diméthylaminosulfonyl)-2-naphthalène carboxylate de méthyle (2.3 g ; 7.13 mmole; 1 eq) dans l'éthanol 50 % avec 428 mg de NaOH (10.71 mmole ; 1.5 eq) pendant 2 h. Ensuite, on évapore sous vide, on reprend dans l'eau, on extrait une fois par l'acétate d'éthyle. On ajoute à la phase aqueuse d'HCl 1N jusqu'à pH 1-2. On extrait 2 fois par le dichlorométhane, on sèche et on évapore.

On obtient 1.92 g de l'acide pur (87 %).
RMN (1H ; DMSO-d₆) : 2.75 (s ; 6H ; N(CH₃)₂), 4.08 (s ; 3H ; OCH₃), 7.74-7.92 (m ; 2H), 8.39 (d, J = 7.9 Hz ; 1H), 8.40 (s ; 1H), 8.65 (d, J = 7.9 Hz ; 1H), 13.57 (s ; 1H ; COOH)

### Stade 3 : 1-Méthoxy-4-(N,N-diméthylaminosulfonyl)-N-[8-(phénylméthyl)-8-azabicyclo[3.2.1]oct-3-β-yl]-2-naphthalène carboxamide chlorhydrate

On additionne 0.4 ml de chloroformiate d'éthyle (3.56 mmole, 1.1 eq) dans 20 ml de dichlorométhane à une solution du 1.0 g de l'acide 1-méthoxy-4-(N,N-diméthylaminosulfonyl)-2-naphthalène carboxylique (3.24 mmole, 1 eq) avec 0.55 ml de la triéthylamine (3.88 mmole, 1.2 eq) dans 70 ml du dichlorométhane à 0°C. Après 15 min, on additionne 0.74 g du 8-(phénylméthyl)-8-azabicyclo[3.2.1]octane-3-β-amine (3.4 mmole, 1.05 eq) dans 20 ml de dichlorométhane à 0°C. On laisse agiter à T.A. jusqu'à la consommation totale des produits de départ. On lave trois par l'eau suivie d'une solution aqueuse saturée de NaCl avant de sécher et d'évaporer. On obtient 1.45 g (88 %) du produit pur après chromatographie Flash sur silice.
RMN (1H; CDCl₃): 1.65-1.87 (m ; 4H); 1.96-2.15 (m ; 4H), 2.93 (s ; 6H ; N(CH₃)2), 3.32 (m ; 2H; H1 et H5), 3.57 (s ; 2H; N-CH₂-Ph) ; 4.00 (s ; 3H ; OCH₃), 4.48 (m ; 1H ; H3) ; 7.22-7.42 (m ; 4H), 7.59-7.78 (m ; 3H), 8.24 (dd, J = 7.7,2.3 ; 1H), 8.63 (s ; 1H), 8.79 (dd, J = 7.7, 2.3 Hz ; 1H)

### Préparation du sel:

On dissout 644 mg de la base obtenu (1.31 mmole; 1 eq) dans 4 ml de méthyléthylcétone. On additionne 0.36 ml d'une solution HCI dans l'isopropanol (3.65 N). On filtre le précipité formé et on sèche.
Rdt.:654 mg (92 %)
Pf: 196 °C
Formule : C₂₈ H₃₄ Cl N₃ O₄ S, 0.1 H₂O
Masse moléculaire: base : 507.66 sel : 545.92

| | % C | % H | % N |
|---|---|---|---|
| Trouvé | 61.78 | 6.38 | 7.53 |
| Calculé | 61.60 | 6.31 | 7.70 |

### Exemple 2 :

### 1,3-Diméthoxy-N-[8-(phénylméthyl)-8-azabicyclo[3.2.1]oct-3-β-yl]-2-naphthalène carboxamide chlorhydrate ( Formule I : R1 = CH₃ ; R2 = 3-OCH₃ ; R3 = H ; Ar = Ph ; n = 1 )

On additionne à une solution de l'acide 1,3-diméthoxy-2-naphthalène carboxylique (1.0 g; 4.31 mmole ; 1 eq) avec quelques gouttes de DMF dans 90 ml du dichlorométhane à 0°C une solution de chlorure d'oxalyle (0.412 ml ; 4.74 mmole ; 1.1 eq) dans 20 ml de dichlorométhane. Ensuite, on laisse agiter à T.A. pendant 1 h.
On additionne cette solution obtenue à une solution du 8-(phénylméthyl)-8-azabicyclo[3.2.1]octane-3-β-amine (0.979 g ; 4.52 mmole ; 1.05 eq) avec 0.72 ml de triéthylamine (5.17 mmole ; 1.2 eq) dans 20 ml de dichlorométhane à 0°C. Ensuite, on laisse agiter à T.A. jusqu'à ce que la réaction soit complète. On lave le mélange réactionnel trois fois par l'eau puis de l'eau saturée de chlorure de sodium. On sèche la phase organique et on évapore. On obtient le produit pur après purification par chromatographie Flash sur silice.

Rdt.: 0.375 g (20 %)
RMN(1H;CDCl₃): 1.67-1.89 (m ; 4H) ; 1.97-2.12 (m ; 4H), 3.31 (m ; 2H ; H1 et H5), 3.60 (s ; 2H ; N-CH₂-Ph) ; 3.88 (s ; 3H ; OCH₃), 3.98 (s ; 3H : OCH3), 4.45 (m ; 1H ; H3), 7.20-749 (m ; 8H) ; 7.68 (d, J = 7.8 Hz ; 1H), 7.99 (d ; J = 7.8 Hz ; 1H)

### Préparation du sel :

On dissout 355 mg de la base obtenu (0.82 mmole ; 1 eq) dans 4 ml de méthyléthylcétone. On additionne 0.23 ml d'une solution HCl dans l'isopropanol (3.65 N). On filtre le précipité formé et on sèche.
Rdt. : 341 mg (88 %)
Pf: : 124°C
Formule : C₂₇ H₃₀ N₂ O₃, 0.9 HCl, 1 H₂O
Masse moléculaire : base : 430.55 sel : 481.38

| | % C | % H | % N |
|---|---|---|---|
| Trouvé | 67.41 | 6.97 | 5.45 |
| Calculé | 67.32 | 6.89 | 5.82 |

### Exemple 3 :

### 1-Méthoxy-N-[8-(phénylméthyl)-8-azabicyclo[3.2.1]oct-3-β-yl]-2-naphthalène carboxamide

De manière analogue à l'exemple 1 au stade 3, mais en partant des réactifs correspondants, le composé du titre est obtenu :
R1 = CH₃
R2=R3=H
C₂₆H₂₈N₂O₂
P.M.=400.53
F°C=125
Ar=Ph
n= 1

| | % C | % H | % N |
|---|---|---|---|
| Trouvé | 77.81 | 7.05 | 6.95 |
| Calculé | 77.97 | 7.05 | 6.99 |

### Exemple 4 :

### 4-Bromo-1-méthoxy-N-[8-(phénylméthyl)-8-azabicyclo[3.2.1 ]oct-3-β-yl]-2-naphthalène carboxamide oxalate

De manière analogue à l'exemple 1 au stade 3, mais en partant des réactifs correspondants, le composé du titre est obtenu :
R1 = CH₃
R2 = 4-Br
R3 = H
C₂₈H₂₉BrN₂O₆
P.M.=569.46
F°C=193
Ar=Ph
n = 1

| | % C | % H | % N |
|---|---|---|---|
| Trouvé | 58.99 | 5.19 | 4.84 |
| Calculé | 58.76 | 5.17 | 4.80 |
| *+ 0.1 | | | |
| AcOEt | | | |

### Exemple 5:

### 4-Chloro-1-méthoxy-N-[8-(phénylméthyl)-8-azabicyclo[3.2.1]oct-3-β-yl]-2-naphthalène carboxamide chlorhydrate

De manière analogue à l'exemple 1 au stade 3, mais en partant des réactifs correspondants le composé du titre est obtenu :
R1 = CH₃
R2 = 4-Cl
R3 = H
C₂₆H₂₉Cl₂N₂O₂
P.M. = 472.44
F°C =203
Ar = Ph
n = 1

| | % C | % H | % N |
|---|---|---|---|
| Trouvé | 66.37 | 6.05 | 5.88 |
| Calculé | 66.07 | 6.14 | 5.93 |

### Exemple 6:

### 1-Méthoxy-4-(N,N-diméthylamino)-N-[8-(phénylméthyl)8-azabicyclo[3.2.1]oct-3-β-yl]-2-naphthalène carboxamide oxalate

De manière analogue à l'exemple 1 au stade 3, mais en partant des réactifs correspondants, le composé du titre est obtenu
R1 = CH₃
R2 = 4-N(CH₃)₂
R3 = H
C₃₁H₃₆N₃O₈
(0.4 H₂O)
(1.5 oxalate)
Ar = Ph
n = 1
P.M. =578.65
(585.85)
F°C=165

| | % C | % H | % N |
|---|---|---|---|
| Trouvé | 63.67 | 6.28 | 7.21 |
| Calculé | 63.53 | 6.33 | 7.17 |

### Exemple 7:

### 4-Amino-1-méthoxy-N-[8-(phénylméthyl)-8-azabicyclo[3.2.1]oct-3-β-yl]-2-naphthalène carboxamide dioxalate

De manière analogue à l'exemple 1 au stade 3, mais en partant des réactifs correspondants, le composé du titre est obtenu.
R1 = CH₃
R2 = 4-NH₂
R3 = H
C₂₆H₂₉N₃O₂,
1.73 HCl
(1.75 H₂O)
Ar = Ph
n = 1
P.M. = 488.45
(510.88)
F°C = 189

| | % C | % H | % N |
|---|---|---|---|
| Trouvé | 61.17 | 6.51 | 8.05 |
| Calculé | 61.13 | 6.75 | 8.22 |

### Exemple 8 :

### 1-Méthoxy-4-nitro-N-[8-(phénylméthyl)-8-azabicyclo[3.2.1]oct-3-β-yl]-2-naphthalène carboxamide chlorhydrate

De manière analogue à l'exemple 1 au stade 3, mais en partant des réactifs correspondants, le composé du titre est obtenu :
R1 = CH₃
R2 = 4-NO₂
R3 = H
C₂₆H₂₈ClN₃O₄
(0.1 H₂O)
Ar = Ph
n= 1
P.M. = 481.98
(483.78)
F°C = 192

| | % C | % H | % N |
|---|---|---|---|
| Trouvé | 64.31 | 5.84 | 8.47 |
| Calculé | 64.55 | 5.88 | 8.69 |

### Exemple 9 :

### 4-Cyano-1-méthoxy-N-[8-(phénylméthyl)-8-azabicyclo[3.2.1]oct-3-β-yl]-2-naphthalène carboxamide chlorhydrate

De manière analogue à l'exemple 1 au stade 3, mais en partant des réactifs correspondants, le composé du titre est obtenu :
R1 = CH₃
R2 = 4-CN
R3 = H
C₂₇H₂₈ClN₃O₂
(0.2 H₂O)
Ar = Ph
n = 1
P.M. = 462.00
(465.59)
F°C = 174

| | % C | % H | % N |
|---|---|---|---|
| Trouvé | 69.88 | 6.17 | 8.94 |
| Calculé | 69.65 | 6.15 | 9.03 |

### Exemple 10 :

### 1,5-Diméthoxy-N-[8-(phénylméthyl)-8-azabicyclo[3.2.1]oct-3-β-yl]-2-naphthalène carboxamide chlorhydrate

De manière analogue à l'exemple 1 au stade 3, mais en partant des réactifs correspondants, le composé du titre est obtenu :
R1 = CH₃
R2 = H
R3 = 5-OCH₃
C₂₇H₃₁ClN₂O₃
(0.14 H₂O)
Ar=Ph
n = 1
P.M. = 466.72
(469.24)
F°C = 216

| | % C | % H | %N |
|---|---|---|---|
| Trouvé | 69.12 | 6.75 | 5.90 |
| Calculé | 69.11 | 6.72 | 5.97 |

### Exemple 11:

### 1,4-Diméthoxy-N-[8-(phénylméthyl)-8-azabicyclo[3.2.1]oct-3-β-yl]-2-naphthalène carboxamide chlorhydrate

De manière analogue à l'exemple 1 au stade 3, mais en partant des réactifs correspondants, le composé du titre est obtenu :
R1 = CH₃
R2 = 4-OCH₃
R3 = H
C₂₇H₃₁ClN₂O₃
P.M. = 467.01
F°C = 216
Ar = Ph
n = 1

| | % C | % H | % N |
|---|---|---|---|
| Trouvé | 69.69 | 6.76 | 5.94 |
| Calculé | 69.44 | 6.69 | 6.00 |

### Exemple 12 :

### 1-Ethoxy-N-[8-(phénylméthyl)-8-azabicyclo[3.2.1]oct-3-β-yl]-2-naphthalène carboxamide chlorhydrate

De manière analogue à l'exemple 1 au stade 3 , mais en partant des réactifs correspondants, le composé du titre est obtenu :
R1 = C₂H₅
R2 = R3 = H
C₂₇H₃₁ClN₂O₂
(0.1 H₂O)
Ar=Ph
n = 1
P.M. = 451.01
(452.81)
F°C = 237

| | % C | % H | % N |
|---|---|---|---|
| Trouvé | 71.56 | 7.04 | 6.06 |
| Calculé | 71.62 | 6.95 | 6.18 |

### Exemple 13 :

### 4-Bromo-1-éthoxy-N-[8-(phénylméthyl)-8-azabicyclo[3.2.1]oct-3-β-yl]-2-naphthalène carboxamide chlorhydrate

De manière analogue à l'exemple 1 au stade 3, mais en partant des réactifs correspondants, le composé du titre est obtenu :
R1 = C₂H₅
R2 = 4-Br
R3=H
C₂₇H₃₀ClBrN₂O₂
P.M. = 529.91
F°C = 215
Ar=Ph
n = 1

| | %C | %H | %N |
|---|---|---|---|
| Trouvé | 61.35 | 5.70 | 5.32 |
| Calculé | 61.20 | 5.71 | 5.29 |

### Exemple 14 :

### 4-Acétamido-1-méthoxy-N-[8-(phénylméthyl)-8-azabicyclo[3.2.1]oct-3-b-yl]-2-naphthalène carboxamide chlorhydrate

De manière analogue à l'exemple 1 au stade 3, mais en partant des réactifs correspondants, le composé du titre est obtenu :
R1 =CH₃
R2 = 4- NHCOCH₃
R3 = H
C₂₈H₃₂ClN₃O₂
(0.2 H₂O)
Ar=Ph
n = 1
P.M.=494.04
(497.63)
F°C = 249

| | % C | % H | % N |
|---|---|---|---|
| Trouvé | 67.87 | 6.66 | 8.25 |
| Calculé | 67.58 | 6.56 | 8.44 |

### Exemple 15 :

### 4-Acétamido-1-méthoxy-N-[8-(4-fluorophénylméthyl)-8-azabicyclo[3.2.1]oct-3-b-yl]-2-naphthalène carboxamide chlorhydrate

De manière analogue à l'exemple 1 au stade 3, mais en partant des réactifs correspondants, le composé du titre est obtenu :
R1 =CH₃
R2 = 4- NHCOCH₃
R3 = H
C₂₈H₃₁FClN₃O₃
(0.1 H₂O)
Ar = 4+F Ph
n = 1
P.M.=494.04
(497.63)
F°C = 249

| | % C | % H | % N |
|---|---|---|---|
| Trouvé | 67.87 | 6.66 | 8.25 |
| Calculé | 67.58 | 6.56 | 8.44 |

### Exemple 16 :

### 4-Acétamido-1-méthoxy-N-[8-(4-chlorophénylméthyl)-8-azabicyclo[3.2.1]oct-3-b-yl]-2-naphthalène carboxamide chlorhydrate

De manière analogue à l'exemple 1 au stade 3, mais en partant des réactifs correspondants, le composé du titre est obtenu :
R1 =CH₃
R2 = 4-NHCOCH₃
R3 = H
C₂₈H₂₁Cl₂N₃O₃
(0.4 H₂O)
Ar = Ph
n = 1
P.M.=528.48
(535.68)
F°C = 255

| | % C | % H | % N |
|---|---|---|---|
| Trouvé | 62.83 | 5.92 | 7.53 |
| Calculé | 62.78 | 5.98 | 7.84 |

### Exemple 17 :

### 4-Amino-1-méthoxy-N-[8-(4-chlorophénylméthyl)-8-azabicyclo[3.2.1]oct-3-b-yl]-2-naphthalène carboxamide dichlorhydrate

De manière analogue à l'exemple 1 au stade 3, mais en partant des réactifs correspondants, le composé du titre est obtenu :
R1 = CH₃
R2 = 4- NH₂
R3 = H
C₂₆H₂₈ClN₃O₂, 1.9 Hcl
(0.5 H₂O)
Ar=4-Cl Ph
n = 1
P.M. = 519.26
(528.26)
F°C = 239

| | % C | % H | % N |
|---|---|---|---|
| Trouvé | 58.90 | 5.78 | 7.85 |
| Calculé | 59.11 | 5.89 | 7.95 |

### Exemple 18 :

### 4-Amino-1-méthoxy-N-[8-(4-fluorophénylméthyl)-8-azabicyclo[3.2.1]oct-3-b-yl]-2-naphthalène carboxamide dichlorhydrate

De manière analogue à l'exemple 1 au stade 3, mais en partant des réactifs correspondants, le composé du titre est obtenu :
R1 = CH₃
R2 = 4-NH₂
R3 = H
C₂₆H₂₈FN₃O₂, 1.95
HCl
(0.6 H₂O)
Ar=4-F Ph
n = 1
P.M. = 504.63
(515.44)
F°C = 236

| | % C | % H | % N |
|---|---|---|---|
| Trouvé | 60.37 | 6.09 | 7.93 |
| Calculé | 60.59 | 6.09 | 8.15 |

### Exemple 19 :

### 4-Amino-1-éthoxy-N-[8-(phénylméthyl)-8-azabicyclo[3.2.1]oct-3-b-yl]-2-naphthalène carboxamide dichlorhydrate

De manière analogue à l'exemple 1 au stade 3, mais en partant des réactifs correspondants, le composé du titre est obtenu :
R1 = C₂H₅
R2 = 4-NH₂
R3 = H
C₂₇H₃₄Cl₂N₃O₂
(1 H₂O)
Ar = Ph
n = 1
P.M. = 503.49
(521.505)
F°C = 230

| | % C | % H | % N |
|---|---|---|---|
| Trouvé | 62.27 | 6.84 | 8.02 |
| Calculé | 62.19 | 6.96 | 8.06 |

### Exemple 20 :

### 1-Ethoxy-4-fluoro-N-[8-(phénylméthyl)-8-azabicyclo[3.2.1]oct-3-b-yl]-2-naphthalène carboxamide

De manière analogue à l'exemple 1 au stade 3, mais en partant des réactifs correspondants, le composé du titre est obtenu :
R1 = C₂H₅
R2 = 4-F
R3 = H
C₂₇H₃₀FClN₂O₂
(0.1 H₂O)
Ar = Ph
n = 1
P.M. = 469.003
(470.804)
F°C = 223

| | %C | %H | %N |
|---|---|---|---|
| Trouvé | 68.86 | 6.43 | 5.86 |
| Calculé | 68.88 | 6.46 | 5.95 |

### Exemple 21 :

### 4-Fluoro-1-méthoxy-N-[8-(phénylméthyl)-8-azabicyclo[3.2.1]oct-3-b-yl]-2-naphthalène carboxamide chlorhydrate

De manière analogue à l'exemple 1 au stade 3, mais en partant des réactifs correspondants, le composé du titre est obtenu :
R1 =CH₃
R2 = 4-F
R3 = H
C₂₆H₂₈FClN₂O₂
(0.1 H₂O)
Ar=Ph
n = 1
P.M.=454.98
(456.77)
F°C = 210

| | % C | % H | % N |
|---|---|---|---|
| Trouvé | 68.25 | 6.21 | 6.03 |
| Calculé | 68.37 | 6.22 | 6.13 |

### Exemple 22 :

### 4-Bromo-1-méthoxy-N-[9-(phénylméthyl)-9-azabicyclo[3.3.1]non-3-b-yl]-2-naphthalène carboxamide

De manière analogue à l'exemple 1 au stade 3, mais en partant des réactifs correspondants, le composé du titre est obtenu :
R1 =CH₃
R2 = 4-Br
R3=H
C₂₆H₂₇BrN₂O₂
P.M.=479.43
F°C = 142
Ar = Ph
n=2

| | % C | % H | % N |
|---|---|---|---|
| Trouvé | 65.57 | 5.97 | 5.67 |
| Calculé | 65.72 | 5.92 | 5.68 |

### Exemple 23 :

### 4-Amino-1-méthoxy-N-[9-(phénylméthyl)-9-azabicyclo[3.3.1]non-3-b-yl]-2-naphthalène carboxamide dichlorhydrate

De manière analogue à l'exemple 1 au stade 3, mais en partant des réactifs correspondants, le composé du titre est obtenu
R1 =CH₃
R2 = 4-NH₂
R3=H
C₂₇H₃₃Cl₂N₃O₂
(1.1 H₂O)
Ar = Ph
n=2
P.M.=502.49
(522.31)
F°C =260

| | % C | % H | % N |
|---|---|---|---|
| Trouvé | 62.25 | 6.92 | 7.93 |
| Calculé | 62.09 | 6.79 | 8.04 |

### Exemple 24 :

### 4-Bromo-1-méthoxy-N-[8-(4-fluorophénylméthyl)-8-azabicyclo[3.2.1]oct-3-b-yl]-2-naphthalène carboxamide oxalate

De manière analogue à l'exemple 1 au stade 3, mais en partant des réactifs correspondants, le composé du titre est obtenu :
R1 =CH₃
R2 = 4-Br
R3 = H
C₂₈H₂₈BrFN₂O₆
(0.5 H₂O)
Ar=4-FPh
n = 1
P.M.=587.45
(596.46)
F°C = 149

| | % C | % H | % N |
|---|---|---|---|
| Trouvé | 56.61 | 4.76 | 4.77 |
| Calculé | 56.38 | 4.90 | 4.69 |

### Exemple 25 :

### 4-Bromo-1-méthoxy-N-[8-(4-chlorophénylméthyl)-8-azabicyclo[3.2.1]oct-3-b-yl]-2-naphthalène carboxamide oxalate

De manière analogue à l'exemple 1 au stade 3, mais en partant des réactifs correspondants, le composé du titre est obtenu :
R1 =CH₃
R2 = 4-Br
R3 = H
C₂₈H₂₈BrClN₂O₆
(0.5 H₂O)
Ar = 4-Cl Ph
n = 1
P.M.=603.91
(612.91)
F°C = 138

| | % C | % H | % N |
|---|---|---|---|
| Trouvé | 54.77 | 4.60 | 4.61 |
| Calculé | 54.87 | 4.77 | 4.57 |

### Exemple 26 :

### 4-Méthylthio-1-méthoxy-N-[8-(phénylméthyl)-8-azabicyclo[3.2.1]oct-3-b-yl]-2-naphthalène carboxamide

De manière analogue à l'exemple 1 au stade 3, mais en partant des réactifs correspondants, le composé du titre est obtenu :
R1 =CH₃
R2 = 4-SCH₃
R3 = H
C₂₇H₃₀N₂O₂S
P.M.=446.61
F°C =104
Ar = Ph
n = 1

| | % C | % H | % N |
|---|---|---|---|
| Trouvé | 72.79 | 6.94 | 6.25 |
| Calculé | 72.91 | 6.77 | 6.27 |

### Exemple 27 :

### 4-Ethylthio-1-méthoxy-N-[8-(phénylméthyl)-8-azabicyclo[3.2.1]oct-3-b-yl]-2-naphthalène carboxamide

De manière analogue à l'exemple 1 au stade 3, mais en partant des réactifs correspondants, le composé du titre est obtenu :
R1 =CH₃
R2 = 4-SC₂H₅
R3 = H
C₂₈H₃₂N₂O₂S
P.M.=460.64
F°C = 82
Ar = Ph
n = 1

| | % C | % H | % N |
|---|---|---|---|
| Trouvé | 73.05 | 7.13 | 5.97 |
| Calculé | 73.01 | 7.00 | 6.08 |

### Exemple 28 :

### 4-Ethylsulfonyl-1-méthoxy-N-[8-(phénylméthyl)-8-azabicyclo[3.2.1]oct-3-b-yl]-2-naphthalène carboxamide

De manière analogue à l'exemple 1 au stade 3, mais en partant des réactifs correspondants, le composé du titre est obtenu :
R1 =CH₃
R2=4-
SO2C2H5
R3 = H
C₂₈H₃₂N₂O₄S
(1.36 H₂O)
Ar = Ph
n = 1
P.M.=492.64
(483.20)
F°C = 119

| | % C | % H | % N |
|---|---|---|---|
| Trouvé | 65.03 | 6.54 | 5.31 |
| Calculé | 65.03 | 6.76 | 5.41 |

### Exemple 29 :

### 4-Aminosulfonyl-1-méthoxy-N-[8-(phénylméthyl)-8-azabicyclo[3.2.1]oct-3-b-yl]-2-naphthalène carboxamide

De manière analogue à l'exemple 1 au stade 3, mais en partant des réactifs correspondants, le composé du titre est obtenu :
R1 =CH₃
R2 = 4-SO₂NH₂
R3 = H
C₂₆H₂₉N₃O₄S
(0.2 H₂O)
Ar = Ph
n = 1
P.M.=479.60
(483.20)
F°C = 199

| | % C | % H | % N |
|---|---|---|---|
| Trouvé | 64.54 | 6.09 | 8.48 |
| Calculé | 64.63 | 6.13 | 8.69 |

### Exemple 30 :

### 4-Hydroxy-1-méthoxy-N-[8-(phénylméthyl)-8-azabicyclo[3.2.1]oct-3-b-yl]-2-naphthalène carboxamide

De manière analogue à l'exemple 1 au stade 3, mais en partant des réactifs correspondants, le composé du titre est obtenu :
R1 =CH₃
R2 = 4-OH
R3 = H
C₂₆H₂₈N₂O₃
(0.1H₂O)
Ar = Ph
n = 1
P.M.=416.52
(418.33)
F°C = 216

| | % C | % H | % N |
|---|---|---|---|
| Trouvé | 74.63 | 6.90 | 6.64 |
| Calculé | 74.65 | 6.79 | 6.69 |

### Exemple 31 :

### 4-Hydroxy-1-éthoxy-N-[8-(phénylméthyl)-8-azabicyclo[3.2.1]oct-3-b-yl]-2-naphthalène carboxamide

R1 = C₂H₅
R2 = 4-OH
R3=H
C₂₇H₃₀N₂O₃
P.M. = 430.55
Ar = Ph
n=1

### Exemple 32 :

### 1-Méthoxy-N-[9-(phénylméthyl)-9-azabicyclo[3.3.1]non-3b-yl]-2-naphthalène carboxamide chlorhydrate

De manière analogue à l'exemple 1 au stade 3, mais en partant des réactifs correspondants, le composé du titre est obtenu :
R1 =CH₃
R2=H
R3 = H
C₂₇H₃₁ClN₂O₂
(0.25H₂O)
Ar=Ph
n = 2
P.M. =451.01
(455.51)
F°C = 232

| | % C | % H | % N |
|---|---|---|---|
| Trouvé | 70.93 | 7.22 | 6.10 |
| Calculé | 71.19 | 6 97 | 6.15 |

### Exemple 33 :

### 1-Ethoxy-4-nitro-N-[8-(phénylméthyl)-8-azabicyclo[3.2.1]oct-3b-yl]-2-naphthalène carboxamide chlorhydrate

De manière analogue à l'exemple 1 au stade 3, mais en partant des réactifs correspondants, le composé du titre est obtenu :
R1 =C₂H₅
R2 =4- NO₂
R3 = H
C₂₇H₃₀ClN₃O₄
P.M.=496.01
F°C = 187
Ar = Ph
n = 1

| | % C | % H | % N |
|---|---|---|---|
| Trouvé | 65.26 | 6.14 | 8.43 |
| Calculé | 65.38 | 6.10 | 8.47 |

## Revendications

1. Un composé de formule générale I : 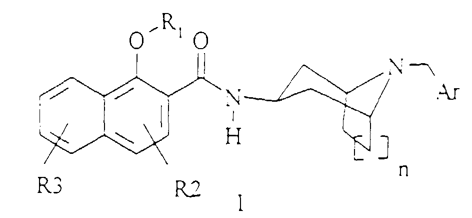
où Aᵣ représente un noyau phényl, le noyau phényl étant ou non substitué par un ou plusieurs substituants choisis parmi, alkyl C₁₋₄, halogène Cl, F, Br, O alkyl C_{1-4,}
n pouvant être un ou deux et former ainsi un 8-azabicyclo[3.2.1]octane ou un 9-azabicyclo[3.3.1]nonane.
Le substituant R₁ étant un groupe alkyl, linéaire ou ramifié en C₁₋₆
Les substituants R₂ et R₃ pouvant être les mêmes ou différents portés par les carbones du cycle aromatique, chacun pouvant être H, Cl, Br, F, alkyl en C₁₋₄, OH, CN, NO₂, S alkyl en C₁₋₄, NH₂, NH alkyl en C₁₋₄, Ndialkyl en C_{1-4,} NHacyl, SO₂NH₂, SO₂Ndialkyl en C₁₋₄, SO₂alkyl en C₁₋₄.
ainsi que les sels d'addition d'acide minéral ou organique pharmaceutiquement acceptable.

2. Un composé de formule générale I selon la revendication 1 caractérisé en ce que R₁ est un méthoxy ou un éthoxy, R₂ est un halogène Cl, Br F, NH₂, NMe₂, OH, Ar un phénol substitué par un halogène Cl, F.

3. Un composé de formule générale I selon les revendications 1 et 2 caractérisé en ce qu'il est choisi parmi les composés suivants et leurs sels d'addition avec un acide minéral ou organique pharmaceutiquement acceptable :
• 1-Méthoxy-4-(N,N-diméthylaminosulfonyl)-N-[8-(phénylméthyl)-8-azabicyclo[3.2.1] oct-3-b-yl]-2-naphthalène carboxamide
• 1,3-Diméthoxy-N-[8-(phénylméthyl)-8-azabicyclo[3.2.1]oct-3-b-yl]-2-naphthalène carboxamide
• 1-Méthoxy-N-[8-(phénylméthyl)-8-azabicyclo[3.2.1]oct-3-b-yl]-2-naphthalène carboxamide
• 4-Bromo-1-méthoxy-N-[8-(phénylméthyl)-8-azabicyclo[3.2.1]oct-3-b-yl]-2-naphthalène carboxamide
• 4-Chloro-1-méthoxy-N-[8-(phénylméthyl)-8-azabicyclo[3.2.1]oct-3-b-yl]-2-naphthalène carboxamide
• 1-Méthoxy-4-(N,N-diméthylamino)-N-[8-(phénylméthyl)8-azabicyclo[3.2.1]oct-3-b-yl]-2-naphthalène carboxamide
• 4-Amino-1-méthoxy-N-[8-(phénylméthyl)-8-azabicyclo[3.2.1]oct-3-b-yl]-2-naphthalène carboxamide
• 1-Méthoxy-4-nitro-N-[8-(phénylméthyl)-8-azabicyclo[3.2.1]oct-3-b-yl]-2-naphthalène carboxamide
• 4-Cyano-1-méthoxy-N-[8-(phénylméthyl)-8-azabicyclo[3.2.1]oct-]-b-yl]-2-naphthalène carboxamide
• 1,5-Diméthoxy-N-[8-(phénylméthyl)-8-azabicyclo[3.2.1]oct-3-b-yl]-2-naphthalène carboxamide
• 1,4-Diméthoxy-N-[8-(phénylméthyl)-8-azabicyclo[3.2.1]oct-3-b-yl]-2-naphthalène carboxamide
• 1-Ethoxy-N-[8-(phénylméthyl)-8-azabicyclo[3.2.1]oct-3-b-yl]-2-naphthalène carboxamide
• 4-Bromo-1-éthoxy-N-[8-(phénylméthyl)-8-azabicyclo[3.2.1]oct-3-b-yl]-2-naphthalène carboxamide
• 4-Acétamido-1-méthoxy-N-[8-(phénylméthyl)-8-azabicyclo[3.2.1]oct-3-b-yl]-2-naphthalène carboxamide
• 4-Acétamido-1-méthoxy-N-[8-(4-fluorophénylmethyl)-8-azabicyclo[3.2.1]oct-3-b-yl]-2-naphthalène carboxamide
• 4-Acétamido-1-méthoxy-N-[8-(4-chlorophénylmethyl)-8-azabicyclo[3.2.1]oct-3-b-yl]-2-naphthalène carboxamide
• 4-Amino-1-méthoxy-N-[8-(4-chlorophénylmethyl)-8-azabicyclo[3.2.1]oct-3-b-yl]-2-naphthaléne carboxamide
• 4-Amino-1-méthoxy-N-[8-(4-fluorophénylmethyl)-8-azabicyclo[3.2.1]oct-3-b-yl]-2-naphthalène carboxamide
• 4-Amino-1-éthoxy-N-[8-(4-fluorophénylméthyl)-8-azabicyclo[3.2.1]oct-3-b-yl]-2-naphthalène carboxamide
• 4-Fluoro-1-éthoxy-N-[8-(phénylméthyl)-8-azabicyclo[3.2.1]oct-3-b-yl]-2-naphthalène carboxamide
• 4-Fluoro-1-méthoxy-N-[8-(phénylméthyl)-8-azabicyclo[3.2.1]oct-3-b-yl]-2-naphthalène carboxamide
• 4-Bromo-1-méthoxy-N-[9-(phénylméthyl)-9-azabicyclo[3.3.1]non-3-b-yl]-2-naphthalène carboxamide
• 4-Amino-1-méthoxy-N-[9-(phénylméthyl)-9-azabicyclo[3.3.1]non-3-b-yl]-2-naphthalène carboxamide
• 4-Bromo-1-méthoxy-N-[8-(4-fluorophénylméthyl)-8-azabicyclo[3.2.1]oct-3-b-yl]-2-naphthalène carboxamide
• 4-Bromo-1-méthoxy-N-[8-(4-chlorophénylméthyl)-8-azabicyclo[3.2.1]oct-3-b-yl]-2-naphthalène carboxamide
• 4-Méthylthio-1-méthoxy-N-[8-(phénylméthyl)-8-azabicyclo[3.2.1]oct-3-b-yl]-2-naphthalène carboxamide
• 4-Ethylthio-1-méthoxy-N-[8-(phénylméthyl)-8-azabicyclo[3.2.1]oct-3-b-yl]-2-naphthalène carboxamide
• 4-Ethylsulfonyl-1-méthoxy-N-[8-(phénylméthyl)-8-azabicyclo[3.2.1]oct-3-b-yl]-2-naphthalène carboxamide
• 4-Aminosulfonyl-1-méthoxy-N-[8-(phénylméthyl)-8-azabicyclo[3.2.1]oct-3-b-yl]-2-naphthalène carboxamide
• 4-Hydroxy-1-méthoxy-N-[8-(phénylméthyl)-8-azabicyclo[3.2.1]oct-3-b-yl]-2-naphthalène carboxamide
• 4-Hydroxy-1-éthoxy-N-[8-(phénylméthyl)-8-azabicyclo[3.2.1]oct-3-b-yl]-2-naphthalène carboxamide
• 1-Méthoxy-N-[9-(phénylméthyl)-9-azabicyclo[3.3.1]non-3b-yl]-2-naphthalène carboxamide
• 1-Ethoxy-4-nitro-N-[8-(phénylméthyl)-8-azabicyclo[3.2.1]oct-3b-yl]-2-naphthalène carboxamide

4. Procédé de préparation du composé de formule générale I selon les revendications 1 à 3 caractérisé en ce que l'on fait réagir un acide naphtalénique de formule II ayant les caractéristiques R₁, R₂, R₃ selon les revendications 1 à 3 avec une amine polycyclique de formule III 3β-amine 8-benzyl 8-azabicyclo[3.2.1]octane ou 3β-amino 9-benzyl 9-azabicyclo[3.3.1]nonane dans les conditions de couplage avec action du chloroformiate d'alkyle en présence de triethylamine dans du chlorure de méthylène à basse température ou par l'intermédiaire du chlorure d'acide de l'acide naphtalénique correspondant.

5. A titre de médicament, les composés de formule I selon l'une des revendications 1 à 3.

6. Composition pharmaceutique caractérisée en ce qu'elle comprend au moins un composé de formule I selon l'une des revendications 1 à 3 et un excipient approprié.

7. Utilisation d'un composé de formule I, selon l'une des revendications 1 à 3, pour la préparation d'un médicament destiné au traitement de la schizophrénie, ses symptômes positifs et négatifs, des désordres obsessionels compulsifs, de l'anxiété, de la dépression, de la toxicomanie, de la dyskinésie tardive et les désordres gastro-intestinaux.

## Patentansprüche

1. Verbindung nach allgemeiner Formel I: 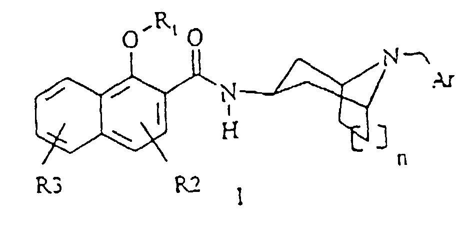
wobei Aᵣ für einen Phenylkern steht, wobei der Phenylkern entweder nicht-substituiert ist oder durch einen oder mehrere Substituenten, ausgewählt unter (C₁₋₄)-Alkyl, Halogen Cl, F, Br und O-(C₁₋₄)-Alkyl, substituiert ist,
wobei n eins oder zwei sein kann und auch ein 8-Azabicyclo[3.2.1]octan oder ein 9-Azabicyclo-[3.3.1]nonan bilden kann,
der Substituent R₁ eine lineare oder verzweigte (C₁₋₆) -Alkylgruppe ist,
die Substituenten R₂ und R₃ gleich oder verschieden sein können, wobei sie von Kohlenstoffen des aromatischen Ringsystems getragen werden, wobei jeder H, Cl, Br, F, (C₁₋₄)-Alkyl, OH, CN, NO₂, S-(C₁₋₄)-alkyl, NH₂, NH- (C₁₋₄)-alkyl, Ndi-(C₁₋₄)-alkyl, NHacyl, SO₂NH₂, SO₂Ndi-(C₁₋₄)-alkyl, SO₂-(C₁₋₄)-alkyl sein kann,
oder auch die Additionssalze mit einer pharmazeutisch akzeptablen Mineralsäure oder einer pharmazeutisch akzeptablen organischen Säure.

2. Verbindung nach der allgemeinen Formel I nach Anspruch 1, dadurch gekennzeichnet, daß R₁ eine Methoxy- oder eine Ethoxygruppe ist, R₂ ein Halogen Cl, Br, F, NH₂, NMe₂, OH ist, Ar ein Phenyl ist, das durch ein Halogen Cl, F substituiert ist.

3. Verbindung nach der allgemeinen Formel I nach den Ansprüchen 1 und 2, dadurch gekennzeichnet, daß sie unter den folgenden Verbindungen und ihren Additionssalzen mit einer pharmazeutisch akzeptablen Mineralsäure oder einer pharmazeutisch akzeptablen organischen Säure ausgewählt ist:
1-Methoxy-4-(N,N-dimethylaminosulfonyl)-N-[8-(phenylmethyl)-8-azabicyclo[3.2.1]oct-3-b-yl]-2-naphthalencarboxamid
1,3-Dimethoxy-N-[8-(phenylmethyl)-8-azabicyclo-[3.2.1]oct-3-b-yl]-2-naphthalencarboxamid
1-Methoxy-N- [8- (phenylmethyl) -8-azabicyclo[3.2.1]oct-3-b-yl]-2-naphthalencarboxamid
4-Bromo-1-methoxy-N-[8-(phenylmethyl)-8-azabicyclo-[3.2.1]oct-3-b-yl]-2-naphthalencarboxamid
4-Chloro-1-methoxy-N-[8-(phenylmethyl)-8-azabicyclo-[3.2.1]oct-3-b-yl]-2-naphthalencarboxamid
1-Methoxy-4-(N,N-dimethylamino)-N-[8-(phenylmethyl)-8-azabicyclo[3.2.1]oct-3-b-yl]-2-naphthalencarboxamid
4-Amino-1-methoxy-N-[8-(phenylmethyl)-8-azabicyclo-[3.2.1]oct-3-b-yl]-2-naphthalencarboxamid
1-Methoxy-4-nitro-N-[8-(phenylmethyl)-8-azabicyclo-[3.2.1]oct-3-b-yl]-2-naphthalencarboxamid
4-Cyano-1-methoxy-N-[8-(phenylmethyl)-8-azabicyclo-(3.2.1]oct-3-b-yl]-2-naphthalencarboxamid
1,5-Dimethoxy-N-[8-(phenylmethyl)-8-azabicyclo-[3.2.1]oct-3-b-yl]-2-naphthalencarboxamid
1,4-Dimethoxy-N-[8-(phenylmethyl)-8-azabicyclo-[3.2.1]oct-3-b-yl]-2-naphthalencarboxamid
1-Ethoxy-N- [8- (phenylmethyl) -8-azabicyclo[3.2.1]oct-3-b-yl]-2-naphthalencarboxamid
4-Bromo-1-ethoxy-N-[8-(phenylmethyl)-8-azabicyclo-[3.2.1]oct-3-b-yl]-2-naphthalencarboxamid
4-Acetamido-1-methoxy-N-[8-(phenylmethyl)-8-azabicyclo[3.2.1]oct-3-b-yl]-2-naphthalencarboxamid
4-Acetamido-1-methoxy-N-[8-(4-fluorophenylmethyl)-8-azabicyclo[3.2.1]oct-3-b-yl]-2-naphthalencarboxamid
4-Acetamido-1-methoxy-N-[8-(4-chlorophenylmethyl)-8-azabicyclo[3.2.1]oct-3-b-yl]-2-naphthalencarboxamid
4-Amino-1-methoxy-N-[8-(4-chlorophenylmethyl)-8-azabicyclo[3.2.1]oct-3-b-yl]-2-naphthalencarboxamid
4-Amino-1-methoxy-N-[8-(4-fluorophenylmethyl)-8-azabicyclo[3.2.1]oct-3-b-yl]-2-naphthalencarboxamid
4-Amino-1-ethoxy-N-[8-(4-fluorophenylmethyl)-8-azabicyclo[3.2.1]oct-3-b-yl]-2-naphthalencarboxamid
4-Fluoro-1-ethoxy-N-[8-(phenylmethyl)-8-azabicyclo-[3.2.1]oct-3-b-yl]-2-naphthalencarboxamid
4-Fluoro-1-methoxy-N-[8-(phenylmethyl)-8-azabicyclo-[3.2.1]oct-3-b-yl]-2-naphthalencarboxamid
4-Bromo-1-methoxy-N-[9-(phenylmethyl)-9-azabicyclo-[3.3.1]non-3-b-yl]-2-naphthalencarboxamid
4-Amino-1-methoxy-N-[9-(phenylmethyl)-9-azabicyclo-[3.3.1]non-3-b-yl]-2-naphthalencarboxamid
4-Bromo-1-methoxy-N-[8-(4-fluorophenylmethyl)-8-azabicyclo[3.2.1]oct-3-b-yl]-2-naphthalencarboxamid
4-Bromo-1-methoxy-N-[8-(4-chlorophenylmethyl)-8-azabicyclo[3.2.1]oct-3-b-yl]-2-naphthalencarboxamid
4-Methylthio-1-methoxy-N-[8-(phenylmethyl)-8-azabicyclo[3.2.1]oct-3-b-yl]-2-naphthalencarboxamid
4-Ethylthio-1-methoxy-N-[8-(phenylmethyl)-8-azabicyclo[3.2.1]oct-3-b-yl]-2-naphthalencarboxamid
4-Ethylsulfonyl-1-methoxy-N-[8-(phenylmethyl)-8-azabicyclo[3.2.1]oct-3-b-yl]-2-naphthalencarboxamid
4-Aminosulfonyl-1-methoxy-N-[8-(phenylmethyl)-8-azabicyclo[3.2.1]oct-3-b-yl]-2-naphthalencarboxamid
4-Hydroxy-1-methoxy-N-[8-(phenylmethyl)-8-azabicyclo-[3.2.1]oct-3-b-yl]-2-naphthalencarboxamid
4-Hydroxy-1-ethoxy-N-[8-(phenylmethyl)-8-azabicyclo-[3.2.1]oct-3-b-yl]-2-naphthalencarboxamid
1-Methoxy-N-[9-(phenylmethyl)-9-azabicyclo[3.3.1]non- 3b-yl]-2-naphthalencarboxamid
1-Ethoxy-4-nitro-N-[8-(phenylmethyl)-8-azabicyclo-[3.2.1]oct-3b-yl]-2-naphthalencarboxamid.

4. Verfahren zur Herstellung einer Verbindung nach der allgemeinen Formel I nach den Ansprüchen 1 bis 3, dadurch gekennzeichnet, daß man eine Naphthalencarbonsäure nach Formel II, die die Charakteristika R₁, R₂, R₃ nach den Ansprüchen 1 bis 3 aufweist, mit einem polycyclischen Amin der Formel III 3β-Amino-8-benzyl-8-azabicyclo[3.2.1]octan oder 3β-Amino-9-benzyl-9-azabicyclo[3.3.1]nonan unter Kupplungsbedingungen mit Einwirkung von Alkylchloroformiat in Gegenwart von Triethylamin in Methylenchlorid bei niedriger Temperatur oder mit Hilfe des Säurechlorids der entsprechenden Naphthalencarbonsäure reagieren läßt.

5. Verbindungen der Formel I nach einem der Ansprüche 1 bis 3 als Arzneimittel.

6. Pharmazeutische Zusammensetzung, dadurch gekennzeichnet, daß sie mindestens eine Verbindung der Formel I nach einem der Ansprüche 1 bis 3 und einen geeigneten Träger umfaßt.

7. Verwendung einer Verbindung der Formel I nach einem der Ansprüche 1 bis 3 zur Herstellung eines Arzneimittels, bestimmt zur Behandlung von Schizophrenie, ihrer positiven und negativen Symptome, kompulsiven Zwangsstörungen, Unruhe, Depression, Toxikomanie, spät eintretender Dyskinesie und gastrointestinalen Störungen.

## Claims

1. Compound of general formula I: 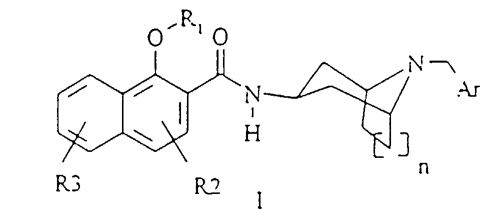
in which Ar represents a phenyl ring, the phenyl ring being unsubstituted or substituted with one or more substituents chosen from C₁₋₄ alkyl, Cl, F or Br halogen or C₁₋₄ O-alkyl,
it being possible for n to be one or two and thus to form an 8-azabicyclo[3.2.1]octane or a 9-azabicyclo-[3.3.1]nonane.
The substituent R₁ being a linear or branched C₁₋₆ alkyl group.
It being possible for the substituents R₂ and R₃, which may be identical or different, to be borne by the carbons of the aromatic ring, each possibly being H, Cl, Br, F, C₁₋₄ alkyl, OH, CN, NO₂, C₁₋₄ S-alkyl, NH₂, C₁₋₄ NH-alkyl, C₁₋₄ N-dialkyl, NH-acyl, SO₂NH₂, C₁₋₄ SO₂N-dialkyl or C₁₋₄ SO₂-alkyl,
as well as the addition salts with a pharmaceutically acceptable inorganic or organic acid.

2. Compound of general formula I according to Claim 1, characterized in that R₁ is a methoxy or an ethoxy, R₂ is a halogen Cl, Br or F, NH₂, NMe₂, OH, Ar or a phenyl substituted with a halogen Cl or F.

3. Compound of general formula I according to Claims 1 and 2, characterized in that it is chosen from the following compounds and the addition salts thereof with a pharmaceutically acceptable inorganic or organic acid:
. 1-methoxy-4-(N,N-dimethylaminosulphonyl)-N-[8-(phenylmethyl)-8-azabicyclo[3.2.1]oct-3-b-yl]-2-naphthalenecarboxamide
. 1,3-dimethoxy-N-[8-(phenylmethyl)-8-azabicyclo[3.2.1]oct-3-b-yl]-2-naphthalene-carboxamide
. 1-methoxy-N-[8-(phenylmethyl)-8-azabicyclo[3.2.1]oct-3-b-yl]-2-naphthalene-carboxamide
. 4-bromo-1-methoxy-N-[8-(phenylmethyl)-8-azabicyclo[3.2.1]oct-3-b-yl]-2-naphthalenecarboxamide
. 4-chloro-1-methoxy-N-[8-(phenylmethyl)-8-azabicyclo[3.2.1]oct-3-b-yl]-2-naphthalenecarboxamide
. 1-methoxy-4-(N,N-dimethylamino)-N-[8-(phenylmethyl)-8-azabicyclo[3.2.1]oct-3-b-yl]-2-naphthalenecarboxamide
. 4-amino-1-methoxy-N-[8-(phenylmethyl)-8-azabicyclo[3.2.1]oct-3-b-yl]-2-naphthalenecarboxamide
. 1-methoxy-4-nitro-N-[8-(phenylmethyl)-8-azabicyclo[3.2.1]oct-3-b-yl]-2-naphthalenecarboxamide
. 4-cyano-1-methoxy-N-[8- (phenylmethyl)-8-azabicyclo[3.2.1]oct-3-b-yl]-2-naphthalenecarboxamide
. 1,5-dimethoxy-N-[8-(phenylmethyl)-8-azabicyclo[3.2.1]oct-3-b-yl]-2-naphthalenecarboxamide
. 1,4-dimethoxy-N-[8-(phenylmethyl)-8-azabicyclo[3.2.1]oct-3-b-yl]-2-naphthalenecarboxamide
. 1-ethoxy-N-[8-(phenylmethyl)-8-azabicyclo[3.2.1]oct-3-b-yl]-2-naphthalenecarboxamide
. 4-bromo-1-ethoxy-N-[8-(phenylmethyl)-8-azabicyclo[3.2.1]oct-3-b-yl]-2-naphthalenecarboxamide
. 4-acetamido-1-methoxy-N-[8-(phenylmethyl)-8-azabicyclo[3.2.1]oct-3-b-yl]-2-naphthalenecarboxamide
. 4-acetamido-1-methoxy-N-[8-(4-fluorophenylmethyl)-8-azabicyclo[3.2.1]oct-3-b-yl]-2-naphthalenecarboxamide
. 4-acetamido-1-methoxy-N-[8-(4-chlorophenylmethyl)-8-azabicyclo[3.2.1]oct-3-b-yl]-2-naphthalenecarboxamide
. 4-amino-1-methoxy-N-[8-(4-chlorophenylmethyl)-8-azabicyclo[3.2.1]oct-3-b-yl]-2-naphthalenecarboxamide
. 4-amino-1-methoxy-N-[8-(4-fluorophenylmethyl)-8-azabicyclo[3.2.1]oct-3-b-yl]-2-naphthalenecarboxamide
. 4-amino-1-ethoxy-N-[8-(4-fluorophenylmethyl)-8-azabicyclo[3.2.1]oct-3-b-yl]-2-naphthalenecarboxamide
. 4-fluoro-1-ethoxy-N-[8-(phenylmethyl)-8-azabicyclo[3.2.1]oct-3-b-yl]-2-naphthalenecarboxamide
. 4-fluoro-1-methoxy-N-[8-(phenylmethyl)-8-azabicyclo[3.2.1]oct-3-b-yl]-2-naphthalenecarboxamide
. 4-bromo-1-methoxy-N-[9-(phenylmethyl)-9-azabicyclo[3.3.1]non-3-b-yl]-2-naphthalenecarboxamide
. 4-amino-1-methoxy-N-[9-(phenylmethyl)-9-azabicyclo[3.3.1]non-3-b-yl]-2-naphthalenecarboxamide
. 4-bromo-1-methoxy-N-[8-(4-fluorophenylmethyl)-8-azabicyclo[3.2.1]oct-3-b-yl]-2-naphthalenecarboxamide
. 4-bromo-1-methoxy-N-[8-(4-chlorophenylmethyl)-8-azabicyclo[3.2.1]oct-3-b-yl]-2-naphthalenecarboxamide
. 4-methylthio-1-methoxy-N-[8-(phenylmethyl)-8-azabicyclo[3.2.1]oct-3-b-yl]-2-naphthalenecarboxamide
. 4-ethylthio-1-methoxy-N-[8-(phenylmethyl)-8-azabicyclo[3.2.1]oct-3-b-yl]-2-naphthalenecarboxamide
. 4-ethylsulphonyl-1-methoxy-N-[8-(phenylmethyl)-8-azabicyclo[3.2.1]oct-3-b-yl]-2-naphthalenecarboxamide
. 4-aminosulphonyl-1-methoxy-N-[8-(phenylmethyl)-8-azabicyclo[3.2.1]oct-3-b-yl]-2-naphthalenecarboxamide
. 4-hydroxy-1-methoxy-N-[8-(phenylmethyl)-8-azabicyclo[3.2.1]oct-3-b-yl]-2-naphthalenecarboxamide
. 4-hydroxy-1-ethoxy-N-[8-(phenylmethyl)-8-azabicyclo[3.2.1]oct-3-b-yl]-2-naphthalenecarboxamide
. 1-methoxy-N-[9-(phenylmethyl)-9-azabicyclo[3.3.1]non-3b-yl]-2-naphthalenecarboxamide
. 1-ethoxy-4-nitro-N-[8-(phenylmethyl)-8-azabicyclo[3.2.1]oct-3b-yl]-2-naphthalenecarboxamide

4. Process for preparing the compound of general formula I according to Claims 1 to 3, characterized in that a naphthenic acid of formula II having the characteristics R₁, R₂ and R₃ according to Claims 1 to 3 is reacted with a polycyclic amine of formula III 3β-amino-8-benzyl-8-azabicyclo[3.2.1]octane or 3β-amino-9-benzyl-9-azabicyclo[3.3.1]nonane under coupling conditions with the action of alkyl chloroformate in the presence of triethylamine in methylene chloride at low temperature, or via the acid chloride of the corresponding naphthenic acid.

5. Compounds of formula I according to one of Claims 1 to 3, as drugs.

6. Pharmaceutical composition, characterized in that it comprises at least one compound of formula I according to one of Claims 1 to 3 and a suitable excipient.

7. Use of a compound of formula I according to one of Claims 1 to 3, for the preparation of a drug intended for the treatment of schizophrenia, its positive and negative symptoms, compulsive obsessive disorders, anxiety, depression, drug addiction, tardive dyskinaesia and gastrointestinal disorders.
